# EUROPEAN PATENT APPLICATION

(11) **EP 2 985 274 A1**
(43) Date of publication of application: **17.02.2016**
(21) Application number: 15154837.7
(22) Date of filing: 12.02.2015
(51) Int. Cl.: C07C 51/09, C07C 51/44, C07C 59/21

(54) **Method for preparation of trifluoroacetylacetic acid**

(30) Priority: 13.08.2014 EP 14180818
(71) Applicant: Lonza Ltd, 3930 Visp (CH)
(72) Inventor: The designation of the inventor has not yet been filed

(57) **Abstract**

The invention discloses a method for the preparation of trifluoroacetylacetic acid.

## Description

The invention discloses a method for the preparation of trifluoroacetylacetic acid.

### BACKGROUND OF THE INVENTION

Trifluoroacetylacetic acid, that is compound of formula (1), is a valuable intermediate for the preparation of 4-alkoxy-1,1,1-trifluorobut-3-en-2-ones of formula (TFB) with R^{TFB} being alkyl, 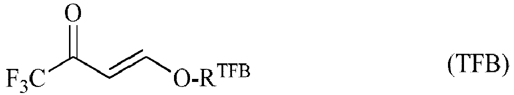 which are important synthetic intermediates for the preparation of fluorinated heterocycles such as trifluoromethylpyridines, which again are intermediates in the production of pharmaceutical, chemical and agro-chemical products, as disclosed for example in WO 2006/059103 A2.

Compound of formula (1) has also been used as intermediate for the preparation of the antiinflammatory drug celecoxib, as disclosed in KR20110001415A of DAE HE CHEMICAL CO., LTD.

Various methods for the preparation of compound of formula (1) and mixtures of compound of formula (1) with its hydrated form, compound of formula (2), 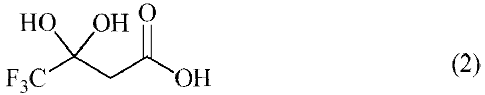 have been reported:
One method is found in F. Swarts, Bulletin de la Classe des Sciences, Academie Royale de Belgique, 1926, 12, 721-725. Swarts teaches the preparation of trifluoroacetoacetic acid by saponification of ethyl trifluoroacetoacetate with aqueous hydrochloric acid. The trifluoroacetoacetic acid was isolated by extraction, to yield mixtures of compound of formula (1) and compound of formula (2). For obtaining the pure dehydrated trifluoroacetoacetic acid (1) an additional dehydration step is reported to be necessary in form of a sublimation process, providing the dehydrated trifluoroacetoacetic acid in low yields. Furthermore, the method disclosed by F. Swarts provides the trifluoroacetoacetic acid with varying amounts of hydrate and no means to determine the amount of hydrated form (2) are disclosed, thereby providing a process difficult to control and of variable results. Swarts mentions sulfuric acid as a substitute for hydrochlorique acid, but stresses the point, that sulfuric acid has the disadvantage that the reaction time is much longer.

Another method is disclosed in KR20110001415A and is a saponification of ethyl trifluoroacetoacetate with sulfuric and trifluoroacetic acid, but no statements about the purity of the product are made. Because the product was extracted from water, it is a mixture of compound of formula (1) and compound of formula (2) as exemplified herein in a comparative example.

Another method is disclosed in US 6,252,105 B1, wherein in Example 11 ethyl alpha,alpha,alpha-trifluoroacetoacetate is converted to trifluoroacetoacetic acid in the presence of trifluoroacetic acid and sulfuric acid. The product is obtained by crystallization. No yield is mentioned, but is presumably low due to the high solubility of trifluoroacetoacetic acid in trifluoroacetic acid. Since the trifluoroacetoacetic acid is obtained by crystallization from a mixture containing sulfuric acid, the product is not free of sulfuric acid, but is contaminated with residual sulfuric acid. To remove this residual sulfuric acid from the product in order to obtain a product which does not contain residual sulfuric acid, a further purification step is necessary and the skilled person knows that this further purification step would be a difficult and thereby cost intensive step, since sulfuric acid cannot be easily separated from the product.

Trifluoroacetic acid is a relatively expensive compound.

There was a need for a simplified process for the preparation of trifluoroacetoacetic acid, which provides the trifluoroacetoacetic acid in its dehydrated form without or at least with only minor amounts of the hydrate of trifluoroacetoacetic acid, which does not need the use of trifluoroacetic acid, and which provides the desired product in high yields. Furthermore there was a need for a method that provides the product with a low content of or with no residual sulfuric acid.

By the method of instant invention compound of formula (1) can be obtained with a low content of compound of formula (2). with a low content of or with no residual sulfuric acid. The method allows for the preparation of compound of formula (1) in large quantities, in industrial scale, in a reproducible way and with high yields.

In the following text, if not otherwise stated, the following meanings are used: ambient pressure usually 1 bar, depending on the weather;

| | |
|---|---|
| alkyl | means a linear or branched alkyl, examples of alkyl include methyl, ethyl, n-propyl, isopropyl, n-butyl, sec-butyl, tert-butyl, pentyl, hexyl, heptyl and the like; |
| alkoxy | means alkyl-O, i.e. the radical obtained by removal of the oxygen-bound hydrogen from an aliphatic alcohol; |
| Ac | acetyl; |
| halogen | means F, Cl, Br or J, preferably F, Cl or Br; |
| hemiacetal | refers to the adduct of an alcohol, for instance methanol or ethanol, with a ketone or with an aldehyde; a hemiacetal may also result upon the addition of water to an enol ether; for instance, the hemiacetal of methanol with 1,1,1-trifluoroacetone is F₃C-C(OH)(OCH₃)-CH₃; |
| hexane | n-hexane, isomeric hexane or mixture of isomeric hexanes; |
| heptane | n-heptane, isomeric heptane or mixture of isomeric heptanes; |
| octane | n-octane, isomeric octane or mixture of isomeric octanes; |
| nonane | n-nonane,. isomeric nonane or mixture of isomeric nonanes; |
| hydrate | refers to the adduct of water with a ketone or with an aldehyde, for instance, the hydrate of 1,1,1-trifluoroacetone is F₃C-C(OH)₂-CH₃; |
| Temp | Temperature; |
| wt-% | percent by weight, weight-%. In case of aqueous ACI the wt-% are based on the combined amount of ACI and water, if not otherwise stated. |

### SUMMARY OF THE INVENTION

Subject of the invention is a method for the preparation of compound of formula (1) with a low content of compound of formula (2); 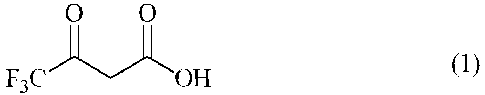 wherein the content of compound of formula (2) is 20% or less, the % are molar percent and are based on the combined molar amount of compound of formula (1) and compound of formula (2);
the method comprises a reaction Reac1 and a separation Separ1;
in Read1 compound of formula (IV) is reacted with compound ACI to provide compound of formula (1); 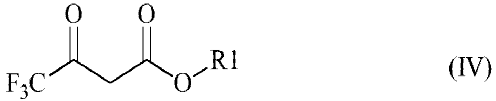
- R1: is C₁₋₈alkyl;
- ACI: is H₂SO₄ or H₃PO₄;
- ACI: is used in form of aqueous ACI;
the content of ACI in the aqueous ACI of from 80 to 99.99 wt-%, the wt-% being based on the combined amount of ACI and water.

Reac1 provides a reaction mixture ReacMix1;

in Separ1 the compound of formula (1) is separated from ReacMix1; Separ1 is a distillation;

Separ1 is done in the presence of a solvent Solv2 and Solv2 is selected from the group consisting of n-hexane, n-heptane, octane, nonane, mixtures of C₅₋₁₀ alkanes, cyclohexane, methylcyclohexane, dimethylcyclohexane, chlorobenzene, 1,2-dichloroethane, 1-chlorobutane, chloroform, carbon tetrachloride, and mixtures thereof.

### DETAILED DESCRIPTION OF THE INVENTION

Preferably, subject of instant invention is a method for preparation of compound of formula (1) with a low content of compound of formula (2), wherein the content of compound of formula (2) is 15% or less, more preferably 10% or less, even more preferably 7.5% or less, especially 5% or less, more especially 2.5% or less, the % are molar percent and are based on the combined molar amount of compound of formula (1) and compound of formula (2).

Preferably, subject of instant invention is a method for preparation of compound of formula (1) with a low content of residual ACI;
more preferably, with less than 10%, even more preferably with less than 5%, especially with less than 2%, more especially with less than 1%, even more especially with less than 0.1%, of residual ACI, the % being % by weight based on the combined weight of the product;
in particular with no residual ACI.

Preferably, no trifluoroacetic acid is used in Reac1;

Preferably, no trifluoroacetic acid is used in Separ1;
more preferably, no trifluoroacetic acid is used in Separ1 and no trifluoroacetic acid is used in Reac1;
even more preferably, no trifluoroacetic acid is used in the method.

Preferably, no water except for the water in the aqueous ACI is used in Reac1;

Preferably, no water except for the water in the aqueous ACI is used in Separ1; more preferably, no water except for the water in the aqueous ACI is used in Separ1 and no
water except for the water in the aqueous ACI is used in Reac1;
even more preferably, no water except for the water in the aqueous ACI is used in the method.

Preferably, R1 is C₁₋₄alkyl;
more preferably, R1 is ethyl.

Preferably, ACI is H₂SO₄.

Preferably, the content of ACI in the aqueous ACI of from 90 to 99.9%, more preferably of from 93 to 99.9%, the wt-% being based on the combined amount of ACI and water.

Preferably, the molar ratio [compound of formula (IV) : ACI] is from [1 : 1] to [1 : 100], more preferably from [1 : 1] to [1 : 50], even more preferably from [1 : 1] to [1 : 30], especially from [1 : 1] to [1 : 20], especially from [1 : 1] to [1 : 15].

Reac1 starts already with mixing of compound of formula (IV) with ACI, and is accelerated by elevation of temperature.

Preferably, the temperature of Reac1 is from 0 to 200 °C, more preferably from 5 to 180 °C, even more preferably from 10 to 160 °C, especially from 15 to 160 °C.

Preferably, Reac1 is done at a pressure of from ambient pressure to 20 bar, more preferably of from ambient pressure to 10 bar, even more preferably of from ambient pressure to 5 bar.

Preferably, the time of Reac1 is from 1 min to 20 h, more preferably from 5 min to 10 h, even more preferably from 5 min to 5 h, especially from 5 min to 4 h.

Preferably, the temperature of Separ1 is from 30 to 200 °C, more preferably from 30 to 180 °C, even more preferably from 35 to 160 °C.

Separ1 can be done at reduced pressure, at ambient pressure or at elevated pressure. Preferably Separ1 is done at reduced pressure or at ambient pressure.

Preferably, Separ1 is done at a pressure of from 0.0001 bar to ambient pressure, more preferably of from 0.001 bar to ambient pressure, even more preferably of from 0.01 bar to ambient pressure.

Preferably, Separ1 is done at such conditions at which ACI does not yet evaporate. These conditions comprise essentially the pressure and the temperature. The relationship between evaporation of ACI on one side and the conditions pressure and temperature on the other side is well known.

Preferably, the time of Separ1 is from 1 min to 20 h, more preferably from 5 min to 10 h, even more preferably from 10 min to 6 h, especially from 20 min to 4 h.

Reac1 and Separ1 can be done in the same devices or can be done in different devices.

When they are done in different devices, the device, in which Reac1 is done, is called the reaction device in the following; and the device, in which Separ1 is done, is called the separation device in the following.

Separ1 can start with, during or after the Reac1; Separ1 can end with Reac1 or can end after Reac1.

Preferably, the temperature of Reac1 and of Separ1 is from 0 to 200 °C, more preferably from 5 to 180 °C, even more preferably from 10 to 160 °C, especially from 15 to 160 °C.

Preferably, Reac1 and Separ1 are done at a pressure of from 0.0001 to 20 bar, more preferably of from 0.001 to 10 bar, even more preferably of from 0.01 to 5 bar.

Preferably, the combined time of Reac1 and of Separ1 is from 5 min to 40 h, more preferably from 10 min to 30 h, even more preferably from 20 min to 20 h, especially from 30 min to 15 h, more especially from 40 min to 10 h.

Reac1 can be done in the presence of a solvent;
the solvent is preferably Solv1 and Solv1 is selected from the group consisting of n-hexane, n-heptane, octane, nonane, mixtures of C₅₋₁₀ alkanes, cyclohexane, methylcyclohexane, dimethylcyclohexane, chlorobenzene, 1,2-dichloroethane, 1-chlorobutane, chloroform, carbon tetrachloride, and mixtures thereof;
more preferable, Solv1 is selected from the group consisting of n-hexane, n-heptane, mixtures of C₅₋₁₀ alkanes, methylcyclohexane, dimethylcyclohexane, chlorobenzene, 1,2-dichloroethane, 1-chlorobutane, chloroform, carbon tetrachloride, and mixtures thereof;
even more preferably, Solv1 is selected from the group consisting of n-hexane, n-heptane, methylcyclohexane, 1,2-dichloroethane, and mixtures thereof.

Preferably, the weight of Solv1 is from 0.1 to 100 times, more preferably from 1 to 50 times, even more preferably from 1 to 40 times, of the weight of compound of formula (IV).

Preferable, Solv2 is selected from the group consisting of n-hexane, n-heptane, mixtures of C₅₋₁₀ alkanes, methylcyclohexane, dimethylcyclohexane, chlorobenzene, 1,2-dichloroethane, 1-chlorobutane, chloroform, carbon tetrachloride, and mixtures thereof;
more preferably, Solv2 is selected from the group consisting of n-hexane, n-heptane, methylcyclohexane, 1,2-dichloroethane, and mixtures thereof.

Preferably, the weight of Solv2 is from 0.1 to 100 times, more preferably from 1 to 50 times, even more preferably from 1 to 40 times, of the weight of compound of formula (IV).

When both Solv1 and Solv2 are used, then preferably the combined weight of Solv1 and Solv2 is from 0.1 to 100 times, more preferably from 1 to 50 times, even more preferably from 1 to 40 times, of the weight of compound of formula (IV).

When Solv1 is used, then preferably Solv1 and Solv2 are identical, which means that preferably Reac1 is done in Solv2.

In case of the use of Solv2, Solv2 can be added to ReacMix1, or ReacMix1 is added to Solv2, or ReacMix1 and Solv2 are added separately to the separation device.

In a preferred embodiment, Reac1 and Separ1 are done in the same device;

In a preferred embodiment, Separ1 starts with or during Reac1, and Reac1 and Separ1 are done in the same device;
more preferably, Separ1 starts during Reac1, and Reac1 and Separ1 are done in the same device;

even more preferably, Separ1 starts during Reac1, Reac1 and Separ1 are done in the same device, and Reac1 is in the presence of Solv2; in this even more preferred embodiment only one solvent is used, that is Solv2, which means that Solv1 and Solv2 are identical.

In another preferred embodiment, Reac1 is done without a solvent.

Preferably, Reac1 and Separ1 are done in different devices;
more preferably, Reac1 and Separ1 are done in different devices, and ReacMix1 and the Solv2 are added separately to the separation device;
even more preferably, Reac1 and Separ1 are done in different devices, ReacMix1 and the Solv2 are added separately to the separation device, and the addition of the Solv2 to the separation device starts at the same time when the addition of ReacMix1 to the separation device starts;
especially, Reac1 and Separ1 are done in different devices, ReacMix1 and the Solv2 are added separately to the separation device, the addition of the Solv2 to the separation device starts at the same time when the addition of ReacMix1 to the separation device starts, and the addition of the Solv2 to the separation device ends after the addition of ReacMix1 to the separation device has ended.

Preferably, Separ1 starts only when the conversion in Reac1 of compound of formula (IV) is 90% or higher, more preferably is 92.5 % or higher, even more preferably is 95% or higher, especially is 97.5% or higher, the % being molar % based on the molar amount of compound of formula (IV).

The separation device is a distillation device.

Preferably, Separ1 is done in the presence of Solv2; also in connection with all the embodiments described herein.

In case that Separ1 is done in the presence of Solv2, a solution SOL of compound of formula (1) in Solv2 is obtained from Separ1. Preferably, compound of formula (1) is isolated from SOL by crystallization.

Compound of formula (IV) are known compounds and can be obtained by known methods, such as disclosed for example in GB 931689 A.

### Examples

In the examples,
the yield of compound of formula (1) is given in % and is a molar yield based on the molar amount of compound of formula (IV), and is determined by NMR spectroscopy;
the amount of compound of formula (2) is given in %, the % are molar percent and are based on the combined molar amount of compound of formula (1) and compound of formula (2), and is determined by NMR spectroscopy;
an internal standard, 1,4-difluorobenzene and sulfolane, for NMR measurement was used to determine the yield and the amount of compound of formula (2);
a statement such as: "The amount of compound of formula (2) is lower than 5%" means that no compound of formula (2) was detected by NMR spectroscopy, and if there was any of compound of formula (2) at all than it was below the detection limit;
if not otherwise stated.

### Example 1

### Compound of formula (4) (1.99 ml, 13.6 mmol),

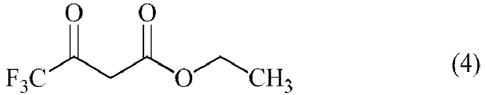

aqueous sulfuric acid (98 wt-%, 5.13 ml, 94.3 mmol), and n-hexane (25 ml) were mixed at ambient pressure and at ambient temperature, after the mixing the resulting mixture was heated with an oil bath to 90°C, the time from mixing until the 90°C were reached was 10 min. Then, within 20 min, the temperature of the oil bath was increased to 110°C, while n-hexane was continuously distilled off and fresh n-hexane (83 ml) was added dropwise to the reaction mixture. Then the reaction mixture was stirred for 2 h at 110°C. ¹H and ¹⁹F NMR of the distillate indicated a yield of 42% of compound of formula (1) in form of a solution in n-hexane.
The amount of compound of formula (2) is lower than 5%.
No residual sulfuric acid was detected in the product.

### Example 2

Example 1 was repeated with two differences: with methylcyclohexane instead of n-hexane and with heating from 90°C to 150°C instead of 90°C to 110°C in the oil bath.

The yield of compound of formula (1) was 35% in form of a solution in methylcyclohexane. The amount of compound of formula (2) is lower than 5%.

No residual sulfuric acid was detected in the product.

### Example 3

Example 1 was repeated with two differences: with n-heptane instead of n-hexane and with heating from 90°C to 140°C instead of 90°C to 110°C in the oil bath.

The yield of compound of formula (1) was 46% in form of a solution in n-heptane.

The amount of compound of formula (2) is lower than 5%.

No residual sulfuric acid was detected in the product.

### Example 4

A mixture of compound of formula (4) (16 ml, 109 mmol) and aqueous sulfuric acid (96 wt-%, 1.08 mol) was stirred at 85°C for 0.5 h. Then the oil bath was heated to 155°C within 20 min. Then during 2 h 1,2-dichloroethane (417 g) was added dropwise continuously, while distilling off volatiles. Four fractions (fractions A, B, C and D) were obtained, which were analyzed by ¹H and ¹⁹F NMR.

**Table 1 shows the content of the four fractions, the percent are the molar percent based on the amount of compound of formula (4) used as substrate.**

| **Table 1** | **1,2-dichloroethane** | **compound (1)** | **compound (4)** |
|---|---|---|---|
| **Fraction A** | 117 g | 7.11 g (42%) | 0.82 g (4.2%) |
| **Fraction B** | 97 g | 1.33 g (7.9%) | |
| **Fraction C** | 96 g | 0.19 g (1.1%) | |
| **Fraction D** | 96 g | 0.03 g (0.2%) | |

The amount of compound of formula (2) is lower than 5%.

No residual sulfuric acid was detected in the product.

### Example 5

A mixture of compound of formula (4) (16 ml, 109 mmol) and aqueous sulfuric acid (96 wt-%, 1.08 mol) was stirred at 60°C for 1 h. Then the mixture was added dropwise within 1.5 h to the distillation flask of a preheated distillation apparatus, the preheating was done with an oil bath having a temperature of 155°C. 1,2-dichloroethane (319 g) was added dropwise to the distillation flask of said distillation apparatus within 2.0 h. The addition of the mixture and if the 1,2-dichloroethane started at the same time. During these additions, volatiles were distilled off, and collected in four fractions (fractions A, B, C and D), which were analyzed by ¹H and ¹⁹F NMR.

**Table 2 shows the content of the four fractions, the percent are the molar percent based on the amount of compound of formula (4) used as substrate.**

| **Table 2** | **1,2-dichloroethane** | **compound (1)** | **compound (4)** |
|---|---|---|---|
| **Fraction A** | 101 g | 3.47 g (21 %) | 0.50 g (11%) |
| **Fraction B** | 113 g | 3.40 g (20%) | 0.16 g (4%) |
| **Fraction C** | 65 g | 2.23 g (13%) | |
| **Fraction D** | 36 g | 0.20 g (1.2%) | |

The amount of compound of formula (2) is lower than 5%.

No residual sulfuric acid was detected in the product.

### Comparative Example 1

Example 2 of KR20110001415A was repeated:

A mixture of ethyl trifluoroacetylacetate (3.65 ml, 25.0 mmol), trifluoroacetic acid (0.974 ml, 12.7 mmol), and sulfuric acid (0.326 ml, 6.0 mmol) was stirred at 90 °C for 3 h. The mixture was then diluted with ethyl acetate (25 ml) and mixed with an aqueous, saturated NaHCO₃ solution (15 ml). The aqueous phase was separated, and the organic phase was extracted with aqueous saturated NaHCO₃ solution (15 ml). The aqueous phases weere combined and were acidified with 2M aqueous hydrochloric acid, and extracted with ethyl acetate (5 times with 5 ml). The combined organic extracts were dried with Na₂SO₄ over night. The drying agent was filtered off, rinsed with AcOEt (2 times with 5 ml), and the combined organic phases were concentrated under reduced pressure (40 °C, 140 to 17 mbar). Obtained was a colorless solid. Analysis by ¹H and ¹⁹F NMR revealed this solid to be mainly the hydrated form of trifluoroacetylacetic acid, which is compound of formula (2), containing less than 10% of the enol form of the water-free trifluoroacetylacetic acid, which is compound of formula (1).

## Claims

1. Method for the preparation of compound of formula (1) with a low content of compound of formula (2); 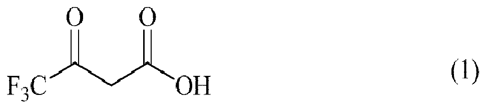 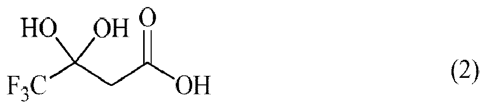 wherein the content of compound of formula (2) is 20% or less, the % are molar percent and are based on the combined molar amount of compound of formula (1) and compound of formula (2);
the method comprises a reaction Reac1 and a separation Separ1;
in Reac1 compound of formula (IV) is reacted with compound ACI to provide compound of formula (1); 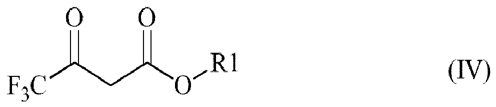
R1 is C₁₋₈ alkyl;
ACI is H₂SO₄ or H₃PO₄;
ACI is used in form of aqueous ACI;
the content of ACI in the aqueous ACI is of from 80 to 99.99 wt-%, the wt-% being based on the combined amount of ACI and water;
Reac1 provides a reaction mixture ReacMix1;
in Separ1 the compound of formula (1) is separated from ReacMix1;
Separ1 is a distillation;
Separ1 is done in the presence of a solvent Solv2 and Solv2 is selected from the group consisting of n-hexane, n-heptane, octane, nonane, mixtures of C₅₋₁₀ alkanes, cyclohexane, methylcyclohexane, dimethylcyclohexane, chlorobenzene, 1,2-dichloroethane, 1-chlorobutane, chloroform, carbon tetrachloride, and mixtures thereof.

2. Method according to claim 1, wherein
R1 is C₁₋₄ alkyl.

3. Method according to claim 1 or 2, wherein
R1 is ethyl.

4. Method according to one or more of claims 1 to 3, wherein
ACI is H₂SO₄.

5. Method according to one or more of claims 1 to 4, wherein
no trifluoroacetic acid is used in Reac1.

6. Method according to one or more of claims 1 to 5, wherein
no trifluoroacetic acid is used in Separ1.

7. Method according to one or more of claims 1 to 6, wherein
Separ1 is done at such conditions at which ACI does not yet evaporate.

8. Method according to one or more of claims 1 to 7, wherein
Reac1 and Separ1 are done in different devices, and ReacMix1 and the Solv2 are added separately to the device in which Separ1 is done.

9. Method according to claim 8, wherein
the addition of the Solv2 to the separation device starts at the same time when the addition of ReacMix1 to the separation device starts.

10. Method according to claim 9, wherein the addition of the Solv2 to the separation device ends after the addition of ReacMix1 to the
separation device has ended.

11. Method according to one or more of claims 1 to 10, wherein Separ1 starts only when the conversion in Reac1 of compound of formula (IV) is 90% or
higher, the % being molar % based on the molar amount of compound of formula (IV).

12. Method according to one or more of claims 1 to 7, wherein
Separ1 starts during Reac1 and Reac1 and Separ1 are done in the same device.

13. Method according to one or more of claims 1 to 12, wherein Reac1 is done in the presence of a solvent.

14. Method according to claim 13, wherein
the solvent is Solv1 and Solv1 is selected from the group consisting of n-hexane, n-heptane, octane, nonane, mixtures of C₅₋₁₀ alkanes, cyclohexane, methylcyclohexane, dimethylcyclohexane, chlorobenzene, 1,2-dichloroethane, 1-chlorobutane, chloroform, carbon tetrachloride, and mixtures thereof.

15. Method according to claim 13, wherein
Reac1 is done in the presence of Solv2.

16. Method according to one or more of claims 1 to 12, wherein
Reac1 is done without a solvent.
